# EUROPEAN PATENT APPLICATION

(11) **EP 4 159 184 A1**
(43) Date of publication of application: **05.04.2023**
(21) Application number: 21200134.1
(22) Date of filing: 30.09.2021
(51) Int. Cl.: A61K 8/02, A61K 8/24, A61K 8/41, A61Q 5/06, A61Q 5/10

(54) **COMPOSITION COMPRISING A DYE AND INORGANIC ALKALIZING AGENT**

(71) Applicant: Kao Corporation, Chuo-ku Tokyo 103-8210 (JP)
(72) Inventor: Nakamura, Takahito, Tokyo, 131-8501 (JP); Hiruma, Daisuke, Tokyo, 131-8501 (JP)
(74) Representative: Hoffmann Eitle

(57) **Abstract**

The present invention is directed to a dyeing composition A for keratin fibers, preferably for human keratin fibers, more preferably for human hair, comprising a) one or more oxidative dye coupler(s) and/or oxidative dye precursor(s), and/or their salt(s), and/or their mixtures, b) one or more salt(s) according to the following structure: X₃PO₄, wherein X is a metal, and wherein the total concentration of water is 10% by weight or less, calculated to the total weight of composition A.

## Description

### Field of the invention

The present invention relates to a composition for dyeing of keratin fibers with an oxidative dye and inorganic alkalizing agent. Furthermore, a dyeing product and dyeing method is disclosed.

### Background of the invention

Oxidative dyes possess superior dyeing ability and grey coverage, but in case of suboptimal storage conditions, their dyeing performance weakens. Especially oxidative dyeing compositions with low or no amount of added water are prone to chemical degradation over storage time.

Cosmetic industry has tackled these problems by excluding air oxygen from the compositions during storage. For example, many dyeing compositions are individually packaged or, if offered as multi-use device, supplied with a protective gas such as nitrogen or carbon dioxide. In addition, formulation parameters of oxidative hair dyes are carefully assessed.

A particular challenge is the stable formulation of dyeing products with low amount of water.

As the prior art has not satisfactorily solved the above challenges, there is a real need to develop dyeing compositions, which are stable over time.

### Summary of the invention

The first object of the present invention is a dyeing composition A for keratin fibers, preferably for human keratin fibers, more preferably for human hair, comprising
a) one or more hair dye(s), preferably one or more oxidative dye coupler(s) and/or oxidative dye precursor(s), and/or their salt(s), and/or their mixtures, and/or one or more direct dye(s), and/or their salt(s), and/or their mixtures,
b) one or more salt(s) according to the following structure

   X₃PO₄

   wherein X is a metal, and
   wherein the total concentration of water is 10% by weight or less, calculated to the total weight of composition A.

The second object of the present invention is a dyeing product for keratin fibers, preferably human keratin fibers, more preferably human hair, comprising:
- composition A as defined in any above,
- a composition B, preferably comprising one or more oxidizing agent(s).

The third object of the present invention is a method for dyeing keratin fibers, preferably human keratin fibers, more preferably human hair, comprising the steps of:
i) mixing composition A as defined above with an aqueous composition, preferably with composition B as defined above to yield a ready-to-use composition having a pH in the range of 7 to 12,
ii) applying the ready-to-use composition onto keratin fibers and leaving it for a time period in the range of 1 min to 60 min,
iii) optionally rinsing-off the keratin fibers and optionally drying the keratin fibers.

### Detailed description of the invention

Inventors of the present invention have unexpectedly found out that composition A according to claim 1 had superior storage stability and dyeing intensity independently of its storage conditions. Furthermore, even after preparation of the ready-to-use mixture, the dyeing performance could be maintained over a certain storage time. The composition also exhibited higher dyeing intensity.

### Composition for dyeing

The present invention is directed to a dyeing composition A for keratin fibers, preferably for human keratin fibers, more preferably for human hair, comprising
a) one or more hair dye(s), preferably one or more oxidative dye coupler(s) and/or oxidative dye precursor(s), and/or their salt(s), and/or their mixtures, and/or one or more direct dye(s), and/or their salt(s), and/or their mixtures,
b) one or more salt(s) according to the following structure

   X₃PO₄

   wherein X is a metal, and
   wherein the total concentration of water is 10% by weight or less, calculated to the total weight of composition A.

Preferably, from the viewpoint of color variations, one or more compound(s) according to group a) is/are p-phenylenediamine, 1,4-diamino-2-methoxymethyl-benzene, p-aminophenol, 2,5-diamino-toluene, 2-N-propyl-p-phenylenediamine, 2-N-ethyl-p-phenylenediamine, 2,6-dimethyl-p-phenylene-diamine, 2-(2,5-diaminophenyl)-ethanol, 1-amino-4-bis-(2'-hydroxyethyl)-amino-benzene, 2-(2-hydroxyethylamino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene, 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 3,5-diamino-1,2,4-triazole, 4-aminophenol and the derivatives thereof such as 4-amino-3-methylphenol, 2-chloro-4-aminophenol, 2,6-dichloro-4-aminophenol, 2,4-diamino-phenol, 2,6-dibromo-4-aminophenol, tetraamino pyrimidines, triaminohydroxy pyrimidines, diaminomono- and -dihydroxy pyrimidines, aminotriazines, 5-amino salicylic acid and/or 1,2,4-triamino benzene, 2,5-diaminopyridine, 2,3-diaminopyridine, 2,6-diaminopyridine, 3-amino-2-methyl amino-6-methoxypyridine, 2-dimethyl-5-aminopyridine, 2-dimethyl aminoethyl-3-hydroxypyridine, 2-amino-4,6-dimethyl pyridine, 2-amino-3-hydroxypyridine, 3-amino-2-(β-hydroxyethyl amino)-6-methoxypyridine, 2,6-dimethyl amino-5-aminopyridine, 2-di(hydroxyethyl) amino-5-aminopyridine, 2-hydroxyethylamino-5-aminopyridine, 4-hydroxy-2,5,6-triaminopyrimidine, and/or their salt(s), and/or their mixtures, more preferably one or more compound(s) according to group a) is/are 1,4-diamino-2-methoxymethyl-benzene, and/or its salt(s).

It is preferred from the viewpoint of stability that one or more compound(s) according to group a) is/are 1,4-diamino-2-methoxymethyl-benzene, and/or its salt(s).

Suitable salts of compound(s) according to group a) is/are sulfate salt, hydrochloride salt, hydrobromide salt, nitrate salt, phosphate salt, hydrogen phosphate salt, dihydrogen phosphate salt, methosulfate salt, citrate salt, succinate salt, tartrate salt, lactate salt, tosylate salt, benzenesulfonate salt, acetate salt, and/or their mixtures, preferably it/they is/are a sulfate salt and/or a hydrochloride salt, and/or their mixtures, more preferably it is a sulfate salt, form the viewpoint of speed of dissolution.

It is preferred from the viewpoint of color brilliance and composition stability that one or more compound(s) according to group a) is/are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9, Disperse Violet 1, Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic Red 51, Basic Yellow 87, HC Blue 17 and Basic Orange 31, Basic Yellow 87, Basic Orange 31, HC Blue 17, Basic Blue 124, HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Blue 18, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Red 18, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, HC Yellow 16, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol, 2-hydroxyethylpicramic acid, an/or their salt(s), and/or their mixtures, preferably one or more compound(s) according to group a) is/are HC Blue 18, HC Red 18, HC Yellow 16, Disperse Black 9, Acid Yellow 1, 2-amino-6-chloro-4-nitrophenol, and/or their salt(s), and/or their mixtures.

Preferably, one or more direct dye(s) may be HC Blue 18, HC Red 18, HC Yellow 16, Disperse Black 9, Acid Yellow 1, 2-amino-6-chloro-4-nitrophenol, and/or their salt(s), and/or their mixtures.

It is preferred from the viewpoint of dyeing intensity that the total concentration of compound(s) according to group a), more preferably the total concentration of 1,4-diamino-2-methoxymethyl-benzene and/or its salt(s), is 0.01% by weight or more, more preferably 0.02% by weight or more, still more preferably 0.03% by weight or more, calculated to the total weight of composition A.

It is preferred from the viewpoint of cosmetic safety that the total concentration of compound(s) according to group a), more preferably the total concentration of 1,4-diamino-2-methoxymethyl-benzene and/or its salt(s), is 65% by weight or less, preferably 50% by weight or less, further more preferably 30% by weight or less, still further more preferably 20% by weight or less, calculated to the total weight of composition A.

For attaining the above-mentioned effects, it is preferred that the total concentration of one or more compound(s) according to group a), more preferably the total concentration of 1,4-diamino-2-methoxymethyl-benzene and/or its salt(s), is/are in the range of 0.01% to 65% by weight, preferably in the range of 0.02% to 50% by weight, more preferably in the range of 0.03% to 30% by weight, more preferably in the range of 0.03% to 20% by weight, calculated to the total weight of composition A.

It is further preferred from the viewpoint of stability that the total concentration of water is 5% by weight or less, preferably 1% by weight or less, more preferably 0.1% by weight or less, calculated to the total weight of composition A, still more preferably composition A is anhydrous.

The term 'anhydrous' denotes a composition that is free of added water. However, this does not exclude bound water such as residual moisture or crystal water.

It is preferred from the viewpoint of solubility that X for compound(s) according to group b) is an alkaline or earth alkaline metal, preferably X is sodium or potassium, more preferably it is sodium.

It is further preferred from the viewpoint of storage stability that compound(s) according to group b) is/are anhydrous compounds.

The most preferred compound according to group b) is/are trisodium phosphate and tripotassium phosphate, in particular anhydrous trisodium phosphate and anhydrous tripotassium phosphate.

It is preferred from the viewpoint of stability and dyeing intensity that the total concentration of compound(s) according to group b), preferably the total concentration of trisodium phosphate, is 1% by weight or more, more preferably 2% by weight or more, further more preferably 5% by weight or more, calculated to the total weight of composition A.

It is preferred from the viewpoint of cosmetic safety that the total concentration of compound(s) according to group b), preferably the total concentration of trisodium phosphate, is 80% by weight or less, more preferably 70% by weight or less, further more preferably 65% by weight or less, calculated to the total weight of composition A.

For attaining the above-mentioned effects, it is preferred that the total concentration of compound(s) according to group b), preferably the total concentration of trisodium phosphate and/or tripotassium phosphate, is in the range 1% to 80% by weight, preferably in the range of 2% to 70% by weight, more preferably in the range of 5% to 65% by weight, calculated to the total weight of composition A.

### Compound(s) according to group c)

It is preferred from the viewpoint of composition stability and convenience of use that composition A comprises one or more pulverulent excipient as compound(s) according to group c).

The term 'excipient' denotes a compound, which may act as filling material and dispersant for the other compounds of composition A and does not react with the dyes or the alkalizing agent, and, thus, confer the powder a high degree of storage stability over an extended period of time.

Composition A of the present invention may comprise an organic and/or an inorganic pulverulent excipient in which dyes are dispersed.

Suitable organic and/or an inorganic pulverulent excipients are, for example, diatomaceous earth, kaolin, bentonite, starch especially corn, tapioca, rice, wheat and potato, nylon powder, montmorillonite, gypsum, sawdust and perlite, more preferably it is corn starch, from the viewpoint of stability.

Preferably, the total concentration of compound(s) according group c), more preferably the total concentration of organic and/or an inorganic pulverulent excipients, is 10% by weight or more, more preferably 15% by weight or more, further more preferably 20% by weight or more, still further more preferably 25% by weight or more, even further more preferably 30% by weight or more, even more preferably 55% by weight or more, calculated to the total weight of composition A, from the viewpoint of achieving good dispersability of the direct dyes in the powder and quick dissolution of the powder.

Preferably, the total concentration of compound(s) according group c), more preferably the total concentration of organic and/or an inorganic pulverulent excipients, is 98% by weight or less, more preferably 95% by weight or less, further more preferably 90% by weight or less, calculated to the total weight of composition A, from the viewpoint of achieving good dispersability of the dyes in the powder and formulation freedom.

For attaining the above mentioned effects, it is preferred that the total concentration of compound(s) according group c), more preferably the total concentration of organic and/or an inorganic pulverulent excipients, is in the range of 10% to 98% by weight, more preferably in the range of 15% to 95% by weight, further more preferably in the range of 20% to 90% by weight, still further more preferably in the range of 25% to 90% by weight, still further more preferably in the range of 30% to 90% by weight, even more preferably in the range of 55% to 90% by weight, calculated to the total weight of composition A.

### Powder composition

In one aspect of the present invention, composition A may be solid composition at 25°C and atmospheric pressure, preferably a powder composition or a pellet composition or a tablet composition or a capsule composition, more preferably a hair dyeing powder composition or a hair dyeing pellet composition or a hair dyeing tablet composition, still more preferably it is a hair dyeing powder composition.

The term 'powder' denotes a solid composition at 25°C and atmospheric pressure. Preferably, form the viewpoint of stability, composition A is an anhydrous powder composition, from the viewpoint of stability.

Suitably, composition A is an anhydrous powder composition, preferably an anhydrous hair dyeing powder composition.

### Lipophilic compounds as compounds according to group d)

Compositions A and/or B may comprise one or more lipophilic compound(s) as compound(s) according to group d).

Preferably, compounds according to group d) are selected from C₁₂ to C₂₂ fatty alcohols, esters of C₃ to C₂₂ alcohols with C₁₂ to C₂₂ fatty acids, C₈ to C₂₂ fatty acids, vegetable oils, and/or silicones, and/or hydrocarbon-based products, and/or their mixtures, from the viewpoint of cosmetic compatibility.

Suitable C₁₂ to C₂₂ fatty alcohols are are myristyl alcohol, cetyl alcohol, stearyl alcohol, behenyl alcohol, and cetearyl alcohol.

Suitable esters of C₃ to C₂₂ alcohols with C₁₂ to C₂₂ fatty acids are isopropyl myristate, isopropyl palmitate, and myristyl myristate.

Suitable C₈ to C₂₂ fatty acids are oleic acid, linoleic acid, and palmitic acid.

Suitable vegetable oils are olive oil, almond oil, sunflower oil, and argan oil.

Suitable silicones are non-aminated and/or aminated silicones. The latter are commonly known as amodimethicones.

It is preferred from the viewpoint of forming a stable composition and user friendliness that the total concentration of compounds according to group d) is 1% by weight or more, more preferably 2% by weight or more, further more preferably 3% by weight or more, calculated to the total weight of each of the compositions A and/or B.

It is preferred from the viewpoint of forming a stable composition that the total concentration of compounds according to group d) is 20% by weight or less, more preferably 15% by weight or less, further more preferably 12% by weight or less, calculated to the total weight of each of the compositions A and/or B.

For attaining the above-mentioned effects, the total concentration of compounds according to group d) is in the range of 1% to 20% by weight, preferably in the range of 2% to 15% by weight, more preferably in the range of 3% to 12% by weight, calculated to the total weight of each of the compositions A and/or B.

### Liquid composition

In another aspect of the present invention, composition A of the present invention is a liquid composition at 25°C and atmospheric pressure comprising one or more organic solvent(s) as compound(s) according to group e), calculated to the total weight of composition A. Preferably, composition A is anhydrous, from the viewpoint of dye stability.

The term 'liquid' denotes a physical state at 25°C and atmospheric pressure, i.e., composition A is liquid at room temperature.

The term 'anhydrous' denotes a composition A, which is free of added water. This does not exclude the presence of residual moisture from air or crystal water bound to ingredients.

For this aspect of the present invention, composition A may comprise one or more organic solvent(s).

The organic solvent(s) may be selected to dissolve the dyes. Preferred solvents are mono-, di-, and trivalent alcohols and/or their mixtures.

Preferred mono-, di-, and trivalent alcohols from the viewpoint of cosmetic safety and dissolution capacity are ethanol, n-propanol, isopropanol, propylene glycol, ethylene glycol, benzyl alcohol, phenoxyethanol, and glycerol, and/or their mixtures.

It is further preferred from the viewpoint of solution stability that the total concentration of organic solvents is 75% by weight or more, more preferably 80% by weight or more, further more preferably 85% by weight or more, calculated to the total weight of composition A.

It is further preferred from the viewpoint of dyeing intensity that the total concentration of organic solvents is 98% by weight or less, more preferably 95% by weight or less, further more preferably 92% by weight or less, calculated to the total weight of composition A.

For attaining the above-mentioned effects, it is preferred that the total concentration of organic solvents is in the range of 75% to 98% by weight, more preferably 80% to 95% by weight, further more preferably in the range of 85% to 92% by weight, calculated to the total weight of composition A.

### Paste-like composition

Optionally, composition A is a paste-like composition, preferably having a viscosity in the range of 10,000 mPas to 500,000 mPas, more preferably in the range of 20,000 mPas to 400,000 mPas, still more preferably in the range of 30,000 mPas to 250,000 mPas, measured by cone-plate viscometry at 25°C and atmospheric pressure, such as a Brookfield viscometer with spindle #4 measured as 10 rpm for 1 min.

For this purpose, composition A may comprise lipophilic compound(s) according to group d) up to a total concentration of 90% by weight.

Preferably, the paste-like composition A is anhydrous, from the viewpoint of dye stability.

### Surfactants as compounds according to f)

Compositions A and/or B may further comprise one or more surfactant(s) as compound according to group f), preferably selected from non-ionic surfactants, anionic surfactants, cationic surfactants, and/or amphoteric/zwitterionic surfactants, and/or their salt(s), and/or their mixtures, more preferably selected from anionic surfactants and/or their salt(s), from the viewpoint of stabilizing the composition and improving wettability and mixability.

Preferably, the anionic surfactants may be selected from ethoxylated or non-ethoxylated alkyl ether sulfate surfactants, alkyl sulfates, ethoxylated and/or non-ethoxylated alkyl carboxylates, ethoxylated or non-ethoxylated amino acid surfactants, and/or their mixtures, and/or their salts.

Suitable examples are alkyl sulfate or preferably ethoxylated alkyl ether sulfate surfactants or mixtures thereof, and/or salts thereof, having an alkyl chain length of C₁₀ to C₂₂ and an ethoxylation degree from 1 to 50.

Suitable non-ionic surfactants may be selected from alkyl polyglycosides, ethoxylated triglycerides, ethoxylated fatty alcohols, ethoxylated fatty acid esters, and/or their mixtures.

Suitable cationic surfactants are quaternary ammonium surfactants having a carbon chain length in the range of C₁₂ to C₂₂ or surfactants having a tertiary amine group and at least one alkyl chain having a carbon chain length in the range of C₁₂ to C₂₂ such as alkylamidoalkylamine surfactants, and/or their salts. Suitable examples are cetrimonium chloride and behentrimonium chloride.

Suitable amphoteric/zwitterionic surfactants are of betaine type. Suitable compounds may be selected from alkyl betaines and/or alkylamido betaines. A preferred compound selected from alkyl betaines is lauryl betaine. A preferred compound selected from alkylamido betaines is cocamidopropyl betaine. The disclosure also relates to the salts of the compounds.

Suitable concentration ranges for surfactants are in the range of 0.1% to 10% by weight, calculated to the total weight of each of the compositions A and/or B, from the viewpoint of enhancing wettability of keratin fibers, physical stability, and mixability with other compositions.

### Thickening polymers

From the viewpoint of cosmetic safety, it is further preferred that the compositions A A and/or B comprise one or more thickening polymer.

Composition A and/or B may comprise one or more thickening polymer(s) selected from non-ionic thickening polymers and/or anionic thickening polymers, and/or their mixtures.

Preferably, the thickening polymers are selected from polymers resulting in an aqueous solution and/or aqueous dispersion at pH ranges between 7 and 12 having a viscosity of at least 1,000 mPa•s measured at a polymer concentration of 1% by weight in water at 25°C determined by cone plate viscometry at 25°C under atmospheric conditions, calculated to the total weight of the composition, determined by cone-plate viscometry such as with a Brookfield viscometer, preferably measured at 10 rpm for 1 min, with an spindle #4.

Suitable non-ionic thickening polymers are cellulose-based polymers. Suitable examples of cellulose-based polymers are methylcellulose, hydroxymethylcellulose, hydroxyethylcellulose, hydroxypropylcellulose, hydroxyethyl-methylcellulose, and alkylated hydroxyl celluloses such as (C₂-C₈)-alkylcelluloses or cetyl hydroxyethylcellulose.

Suitable anionic thickening polymers are selected from naturally-based anionic polymers and/or synthetic anionic polymers.

Suitably, the natural anionic polymer(s) may be selected from xanthan gum, dehydroxanthan gum, hydroxypropylxanthan gum, carboxymethyl cellulose and starch based polymers such as vegetable starch and/or their synthetically modified derivatives such as hydroxypropyl starch phosphate. Equally suitable are alginic acids, sodium alginates, ammonium alginates, calcium alginates, gum arabic, and guar gum.

Suitable synthetic anionic polymers are associative thickening polymers, such as acrylates/steareth-30 methacrylate copolymer.

The preferred thickening polymer for the composition of the present invention are natural anionic polymers, more preferably xanthan gum and/or dehydroxanthan gum, from the viewpoint of their biodegradability and low environmental impact.

Preferably, the total concentration of thickening polymers in compositions A and/or B is 0.1% by weight or more, more preferably 0.25% by weight or more, more preferably 0.5% by weight or more, calculated to the total weight of each of the compositions A, and/or B, from the viewpoint of providing sufficient viscosity to the composition.

Preferably, the total concentration of thickening polymers in compositions A and/or B is 15% by weight or less, more preferably 12% by weight or more, further more preferably 10% by weight or less, calculated to the total weight of each of the compositions A and/or B, from the viewpoint of providing sufficient viscosity to the composition and cost of goods.

For attaining the above-mentioned effects, it is preferred that the total concentration of thickening polymers in compositions A and/or B is in the range of 0.1% to 15% by weight, preferably in the range of 0.25% to 12% by weight, more preferably in the range of 0.5% to 10% by weight, calculated to the total weight of each of the compositions A and/or B.

It is preferred from the viewpoint of cosmetic safety that the composition of the present invention, in case that it is aqueous, has a viscosity in the range of 1,000 Pas to 25,000 mPas, preferably 2,000 mPas to 20,000 mPas, more preferably in the range of 2,500 mPas to 17,500 mPas, determined by cone plate viscometry at 25°C under atmospheric conditions such as measured with a Brookfield viscometer with spindle #4 at 10 rpm for 1 min.

### Oxidizing agents

Compositions A and/or B may comprise one or more oxidizing agent(s).

Preferably, composition A comprises one or more oxidizing agent being solid at 25°C and atmospheric pressure, more preferably preferably percarbonates, borates, perborates, melamine peroxide, urea peroxide, and/or their salt(s), and/or their mixtures.

Suitable concentrations for oxidizing agents in composition A are in the range of 1% to 50% by weight, preferably in the range of 5% to 45% by weight, calculated to the total weight of composition A.

The preferred oxidizing agent for composition B is hydrogen peroxide, from the viewpoint of dyeing performance and hair damage.

It is further preferred from the viewpoint of dyeing performance that the concentration of oxidizing agents in the composition B is 0.25% by weight or more, more preferably 0.5% by weight or more, further more preferably 1% by weight or more, calculated to the total weight of composition B.

It is further preferred from the viewpoint of product performance and user safety that the concentration oxidizing agents in composition B is 30% by weight or less, more preferably 25% by weight or less, calculated to the total weight of composition B.

For attaining the above-mentioned effects, it is preferred that the concentration of oxidizing agents in composition B is in the range of 0.25% to 30% by weight, more preferably in the range of 0.5% to 25% by weight, still more preferably in the range of 1% to 25% by weight, calculated to the total weight of composition B.

### Dyeing product

The present invention is also directed to a dyeing product for keratin fibers, preferably human keratin fibers, more preferably human hair, comprising:
- composition A as defined above,
- a composition B.

Preferably, composition B is an aqueous composition. The term 'aqueous' denotes a composition comprising 50% by weight of water or more, calculated to the total weight of the composition.

Preferably, from the viewpoint of stability, the composition B has a pH in the range of 1 to 7, preferably in the range of 1.5 to 5, further more preferably in the range of 2 to 4.5

It is further preferred from the viewpoint of cosmetic safety that one or more oxidizing agent(s) of composition B is hydrogen peroxide.

Suitably, the pH is measured with a glass electrode at 25°C under atmospheric conditions.

It is further preferred from the viewpoint of dyeing performance that the concentration of oxidizing agents, preferably the concentration of hydrogen peroxide, in the composition B is 0.25% by weight or more, more preferably 0.5% by weight or more, further more preferably 1% by weight or more, calculated to the total weight of composition B.

It is further preferred from the viewpoint of product performance and user safety that the concentration oxidizing agents, preferably the concentration of hydrogen peroxide, in the composition B is 30% by weight or less, more preferably 25% by weight or less, calculated to the total weight of composition B.

For attaining the above-mentioned effects, it is preferred that the concentration of oxidizing agents, preferably the concentration of hydrogen peroxide, in the composition B is in the range of 0.25% to 30% by weight, more preferably in the range of 0.5% to 25% by weight, still more preferably in the range of 1% to 25% by weight, calculated to the total weight of composition B.

Composition B preferably comprises one or more alkylamine salt(s) or alkanolamine salt(s), preferably according to the following general structure 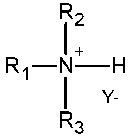 wherein R1, R2, and R3 are independently selected from H, linear C1-C6 alkyl which may be substituted with one hydroxyl group, or branched C3-C12 alkyl or alkanol, wherein at least one of R1, R2, or R3 is different from H, and Y- is an anion, from the viewpoint of dyeing intensity.

Preferably, from the viewpoint of solubility, the anion Y- is sulfate, hydrochloride, chloride, hydrobromide, bromide, nitrate, phosphate, hydrogen phosphate, dihydrogen phosphate, methosulfate, citrate, succinate, tartrate, lactate, tosylate, benzoate, benzenesulfonate, acetate, and/or their mixtures

It is further preferred from the viewpoint of commercial availability that one or more alkylamine salt(s) or alkanolamine salt(s) is/are salts of mono- and/or diethanolamine, butyl ethanolamine, butyl diethanolamine, dibutyl ethanolamine, methylethanolamine, triethanolamine, N-lauryl diethanolamine, diisopropanolamine, dimethyl isopropanolamine, isopropanolamine, triisopropanolamine, isobutanolamine, tris-(hydroxymethyl)-aminomethane, and/or their mixtures, more preferably it/they is/are mono- and/or diethanolamine salt(s), and/or their mixtures.

It is preferred from the viewpoint of wash fastness that the total concentration of alkylamine salt(s) or alkanolamine salt(s) is/are 0.1% by weight or more, more preferably 0.2% by weight or more, further more preferably 0.5% by weight or more, calculated to the total weight of compositions B.

It is preferred from the viewpoint of cosmetic safety that the total concentration of alkylamine salt(s) or alkanolamine salt(s) is/are 15% by weight or less, more preferably 12% by weight or less, further more preferably 10% by weight or less, still more preferably 8% by weight or less, calculated to the total weight of composition B.

For attaining the above-mentioned effects, it is preferred that the total concentration of alkylamine salt(s) or alkanolamine salt(s) is in the range 0.1% to 15% by weight, preferably in the range of 0.2% to 12% by weight, more preferably in the range of 0.5% to 10% by weight, more preferably in the range of 0.5% to 8% by weight, calculated to the total weight of composition B.

Composition B preferably is an emulsion, thickened gel, or a combination thereof, from the viewpoint of cosmetic safety as well as user friendliness. It may comprise lipophilic compound(s) according to group d) and/or surfactant(s) according to group f) and/or one or more thickening polymer(s) as defined above.

It is preferred from the viewpoint of forming a stable composition and user friendliness that the total concentration of compounds according to group d) is 1% by weight or more, more preferably 2% by weight or more, further more preferably 3% by weight or more, calculated to the total weight of composition B.

It is preferred from the viewpoint of forming a stable composition that the total concentration of compounds according to group d) is 20% by weight or less, more preferably 15% by weight or less, further more preferably 12% by weight or less, calculated to the total weight of composition B.

For attaining the above-mentioned effects, the total concentration of compounds according to group d) is in the range of 1% to 20% by weight, preferably in the range of 2% to 15% by weight, more preferably in the range of 3% to 12% by weight, calculated to the total weight of composition B.

Composition B may further comprise one or more surfactant(s) as compound according to group f), as defined above for the composition A of the present invention above.

Suitable concentration ranges for surfactants are in the range of 0.1% to 10% by weight, calculated to the total weight of composition B, from the viewpoint of enhancing wettability of keratin fibers, physical stability, and mixability with other compositions.

From the viewpoint of cosmetic safety, it is further preferred that composition B comprises one or more thickening polymer, as defined for the composition of the present invention above.

Preferably, the total concentration of thickening polymers in composition B is 0.1% by weight or more, more preferably 0.25% by weight or more, more preferably 0.5% by weight or more, calculated to the total weight of composition B, from the viewpoint of providing sufficient viscosity to composition B.

Preferably, the total concentration of thickening polymers in composition B is 15% by weight or less, more preferably 12% by weight or more, further more preferably 10% by weight or less, calculated to the total weight of composition B, from the viewpoint of providing sufficient viscosity to composition B and cost of goods.

For attaining the above-mentioned effects, it is preferred that the total concentration of thickening polymers in composition B is in the range of 0.1% to 15% by weight, preferably in the range of 0.25% to 12% by weight, more preferably in the range of 0.5% to 10% by weight, calculated to the total weight of composition B.

It is preferred from the viewpoint of cosmetic safety that composition B has a viscosity in the range of 1,000 Pas to 25,000 mPas, preferably 2,000 mPas to 20,000 mPas, more preferably in the range of 2,500 mPas to 17,500 mPas, determined by cone plate viscometry at 25°C under atmospheric conditions. A suitable viscometer is a Brookfield viscometer with spindle #4 and viscosity is measured at 10 rpm for 1 min.

It is further preferred from the viewpoint of stability of the ready-to-use mixture that the mixing ratio by weight of compositions A and B is 1:1 or more, preferably 1:2 or more, more preferably 1:4 or more, still more preferably 1:5 or more.

It is further preferred from the viewpoint of stability of the ready-to-use mixture and cosmetic appearance that the mixing ratio by weight of compositions A and B is 1:20 or less, preferably 1:15 or less, more preferably 1:12 or less, still more preferably 1:10 or less.

For attaining the above-mentioned effects, it is preferred that the mixing ratio by weight of compositions A and B is in the range of 1:1 to 1:20, preferably in the range of 1:2 to 1:15, more preferably in the range of 1:4 to 1:12, still more preferably in the range of 1:5 to 1:10

### Method for dyeing

The present invention is also directed to a method for dyeing keratin fibers, preferably human keratin fibers, more preferably human hair, comprising the steps of:
i) mixing composition A as defined above with an aqueous composition, preferably with composition B as defined above to yield a ready-to-use composition having a pH in the range of 7 to 12,
ii) applying the ready-to-use composition onto keratin fibers and leaving it for a time period in the range of 1 min to 60 min,
iii) optionally rinsing-off the keratin fibers and optionally drying the keratin fibers. If composition A comprises oxidizing agents, then composition B may be free of oxidizing agents.

The ready-to-use composition is applied to keratin fibers and preferably left for a time period of 1 min to 60 min as defined in step ii). Further preferred time ranges for step ii) are 5 min to 45 min, more preferred ranges are 10 min to 35 min, from the viewpoint of sufficiently dyeing.

Optionally, heat may be applied while leaving the composition A or the ready-to-use composition onto keratin fibers. Suitable temperature ranges are 30°C to 50°C.

Preferably, the mixing ratio by weight of compositions A and B in step i) is in the range of 1:1 to 1:20, preferably in the range of 1:2 to 1:15, more preferably in the range of 1:4 to 1:12, still more preferably in the range of 1:5 to 1:10.

Thus, the disclosure of the present invention also relates to a method for dyeing keratin fibers, preferably human keratin fibers, more preferably human hair, comprising the steps of:
i) mixing composition A as defined above with an aqueous composition B comprising hydrogen peroxide, to yield a ready-to-use composition having a pH in the range of 7 to 12,
ii) applying the ready-to-use composition onto keratin fibers and leaving it for a time period in the range of 1 min to 60 min,
iii) optionally rinsing-off the keratin fibers and optionally drying the keratin fibers,
wherein the mixing ratio by weight of compositions A and B in step i) is in the range of 1:1 to 1:20, preferably in the range of 1:2 to 1:15, more preferably in the range of 1:4 to 1:12, still more preferably in the range of 1:5 to 1:10.

The present disclosure is also directed to <1> a dyeing composition A for keratin fibers, preferably for human keratin fibers, more preferably for human hair, comprising
a) one or more hair dye(s), preferably one or more oxidative dye coupler(s) and/or oxidative dye precursor(s), and/or their salt(s), and/or their mixtures, and/or one or more direct dye(s), and/or their salt(s), and/or their mixtures,
b) one or more salt(s) according to the following structure

   X₃PO₄

   wherein X is a metal, and
   wherein the total concentration of water is 10% by weight or less, calculated to the total weight of composition A.

<2> The composition according to clause <1> characterized in that one or more compound(s) according to group a) is/are p-phenylenediamine, 1,4-diamino-2-methoxymethyl-benzene, p-aminophenol, 2,5-diamino-toluene, 2-N-propyl-p-phenylenediamine, 2-N-ethyl-p-phenylenediamine, 2,6-dimethyl-p-phenylene-diamine, 2-(2,5-diaminophenyl)-ethanol, 1-amino-4-bis-(2'-hydroxyethyl)-aminobenzene, 2-(2-hydroxyethylamino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene, 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 3,5-diamino-1,2,4-triazole, 4-aminophenol and the derivatives thereof such as 4-amino-3-methylphenol, 2-chloro-4-aminophenol, 2,6-dichloro-4-aminophenol, 2,4-diamino-phenol, 2,6-dibromo-4-aminophenol, tetraamino pyrimidines, triaminohydroxy pyrimidines, diaminomono- and -dihydroxy pyrimidines, aminotriazines, 5-amino salicylic acid and/or 1,2,4-triamino benzene, 2,5-diaminopyridine, 2,3-diaminopyridine, 2,6-diaminopyridine, 3-amino-2-methyl amino-6-methoxypyridine, 2-dimethyl-5-aminopyridine, 2-dimethyl aminoethyl-3-hydroxypyridine, 2-amino-4,6-dimethyl pyridine, 2-amino-3-hydroxypyridine, 3-amino-2-(β-hydroxyethyl amino)-6-methoxypyridine, 2,6-dimethyl amino-5-aminopyridine, 2-di(hydroxyethyl) amino-5-aminopyridine, 2-hydroxyethylamino-5-aminopyridine, 4-hydroxy-2,5,6-triaminopyrimidine, and/or their salt(s), and/or their mixtures, preferably one or more compound(s) according to group a) is/are 1,4-diamino-2-methoxymethyl-benzene, and/or its salt(s).

<3> The composition according to any of the clauses <1> to <2> characterized in that one or more salt(s) of compound(s) according to group a) is/are sulfate salt, hydrochloride salt, hydrobromide salt, nitrate salt, phosphate salt, hydrogen phosphate salt, dihydrogen phosphate salt, methosulfate salt, citrate salt, succinate salt, tartrate salt, lactate salt, tosylate salt, benzenesulfonate salt, acetate salt, and/or their mixtures, preferably it/they is/are a sulfate salt and/or a hydrochloride salt, and/or their mixtures, more preferably it is a sulfate salt.

<4> The composition according to any of the clauses <1> to <3> characterized in that one or more compound(s) according to group a) is/are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9, Disperse Violet 1, Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic Red 51, Basic Yellow 87, HC Blue 17 and Basic Orange 31, Basic Yellow 87, Basic Orange 31, HC Blue 17, Basic Blue 124, HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Blue 18, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Red 18, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, HC Yellow 16, 2-Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol, 2-hydroxyethylpicramic acid, an/or their salt(s), and/or their mixtures, preferably one or more compound(s) according to group a) is/are HC Blue 18, HC Red 18, HC Yellow 16, Disperse Black 9, Acid Yellow 1, 2-amino-6-chloro-4-nitrophenol, and/or their salt(s), and/or their mixtures.

<5> The composition according to any of the clauses <1> to <4> characterized in that the total concentration of compound(s) according to group a), preferably the total concentration of 1,4-diamino-2-methoxymethyl-benzene, and/or its salt(s), is in the range of 0.01% to 65% by weight, preferably in the range of 0.02% to 50% by weight, more preferably in the range of 0.03% to 30% by weight, more preferably in the range of 0.03% to 20% by weight, calculated to the total weight of composition A.

<6> The composition according to any of the clauses <1> to <5> characterized in that X for compound(s) according to group b) is an alkaline or earth alkaline metal, preferably X is sodium or potassium, more preferably it is sodium.

<7> The composition according to any of clauses <1> to <6> characterized in that compound(s) according to group b) is/are anhydrous compounds.

<8> The composition according to any of the clauses <1> to <7> characterized in that the total concentration of compound(s) according to group b), preferably the total concentration of trisodium phosphate and/or tripotassium phosphate, is in the range of 1% to 80% by weight, preferably in the range of 2% to 70% by weight, more preferably in the range of 5% to 65% by weight, calculated to the total weight of composition A.

<9> The composition according to any of the clauses <1> to <8> characterized in that the total concentration of water in composition A is 5% by weight or less, preferably 1% by weight or less, more preferably 0.1% by weight or less, calculated to the total weight of composition A, still more preferably composition A is an anhydrous composition.

<10> The composition according to any of the clauses <1> to <9> characterized in that it comprises one or more direct dye(s), preferably HC Blue 18, HC Red 18, HC Yellow 16, Disperse Black 9, Acid Yellow 1, 2-amino-6-chloro-4-nitrophenol, and/or their salt(s), and/or their mixtures.

<11> The composition according to clause <10> characterized in that the total concentration of direct dyes in composition A is in the range of 0.01% to 20% by weight is in the range of 0.05% to 15% by weight, more preferably in the range of 0.1% to 10% by weight, calculated to the total weight of composition A.

<12> The composition according to any of the clauses <1> to <11> characterized in that it comprises one or more pulverulent excipient as compound(s) according to group c).

<13> The composition according to clause <12> characterized in that it comprises an organic and/or an inorganic pulverulent excipient as compound(s) according to group c).

<14> The composition according to any of the clauses <12> to <13> characterized in that one or more compound(s) according to group c) is/are diatomaceous earth, kaolin, bentonite, starch especially corn, tapioca, rice, wheat and potato, nylon powder, montmorillonite, gypsum, sawdust and perlite, more preferably it is corn starch, and/or their mixtures.

<15> The composition according to any of the clauses <12> to <14> characterized in that the total concentration of compound(s) according group c), more preferably the total concentration of organic and/or an inorganic pulverulent excipients, is in the range of 10% to 98% by weight, more preferably in the range of 15% to 95% by weight, further more preferably in the range of 20% to 90% by weight, still further more preferably in the range of 25% to 90% by weight, still further more preferably in the range of 30% to 90% by weight, even more preferably in the range of 55% to 90% by weight, calculated to the total weight of composition A.

<16> The composition according to any of the clauses <12> to <15> characterized in that it is a solid composition at 25°C and atmospheric pressure, preferably a powder composition or a pellet composition or a tablet composition or a capsule composition, more preferably a hair dyeing powder composition or a hair dyeing pellet composition or a hair dyeing tablet composition, still more preferably it is a hair dyeing powder composition.

<17> The composition according to clause <16> characterized in that it is an anhydrous powder composition, preferably an anhydrous hair dyeing powder composition.

<18> The composition according to any of the clauses <1> to <17> characterized in that it comprises one or more lipophilic compound(s) as compound(s) according to group d), preferably selected from C₁₂ to C₂₂ fatty alcohols, esters of C₃ to C₂₂ alcohols with C₁₂ to C₂₂ fatty acids, C₈ to C₂₂ fatty acids, vegetable oils, and/or silicones, and/or hydrocarbon-based products, and/or their mixtures.

<19> The composition according to clause <18> characterized in that one or more C₁₂ to C₂₂ fatty alcohol(s) as compound(s) according to group d) is/are myristyl alcohol, cetyl alcohol, stearyl alcohol, behenyl alcohol, and cetearyl alcohol, one or more esters of C₃ to C₂₂ alcohols with C₁₂ to C₂₂ fatty acids as compound(s) according to group d) is/are isopropyl myristate, isopropyl palmitate, and myristyl myristate, one or more C₈ to C₂₂ fatty acids as compound(s) according to group d) is/are oleic acid, linoleic acid, and palmitic acid, one or more vegetable oil(s) as compound(s) according to group d) is/are olive oil, almond oil, sunflower oil, and argan oil, one or more silicone(s) as compound(s) according to e) is/are non-aminated and/or aminated silicones, and/or their mixtures.

<20> The composition according to any of the clauses <18> to <19> characterized in that the total concentration of compounds according to group d) is in the range of 1% to 20% by weight, preferably in the range of 2% to 15% by weight, more preferably in the range of 3% to 12% by weight, calculated to the total weight of composition A.

<21> The composition according to any of the clauses <1> to <15> and/or <18> to <20> characterized in that it is a hair dyeing oil.

<22> The composition according to clause <21> characterized in that it has a paste-like viscosity, more preferably it has a viscosity in the range of 10,000 mPas to 500,000 mPas, more preferably in the range of 20,000 mPas to 400,000 mPas, still more preferably in the range of 30,000 mPas to 250,000 mPas, measured by cone-plate viscometry at 25°C and atmospheric pressure.

<23> The composition according to any of the clauses <21> to <22> characterized in that the total concentration of one or more compound(s) according to group d) is 90% by weight or less, preferably in the range of 50% to 90% by weight, calculated to the total weight of composition A.

<24> The composition according to any of the clauses <1> to <11> characterized in that is a liquid composition at 25°C and atmospheric pressure and comprises one or more organic solvent(s) as compound(s) according to group e), preferably one or more mono-, di-, and trivalent alcohols, and/or their mixtures as one or more compound(s) according to e).

<25> The composition according to clause <24> characterized in that one or more compound(s) according to group e) is/are ethanol, n-propanol, isopropanol, propylene glycol, ethylene glycol, benzyl alcohol, phenoxyethanol, and glycerol, and/or their mixtures.

<26> The composition according to any of the clauses <24> to <25> characterized in that the total concentration of one or more compound(s) according to group e) is in the range of 75% to 98% by weight, more preferably 80% to 95% by weight, further more preferably in the range of 85% to 92% by weight, calculated to the total weight of composition A.

<27> The composition according to any of the clauses <1> to <26> characterized in that it comprises one or more surfactant(s) as compound according to group f), preferably selected from non-ionic surfactants, anionic surfactants, cationic surfactants, and/or amphoteric/zwitterionic surfactants, and/or their salt(s), and/or their mixtures, more preferably selected from anionic surfactants and/or their salt(s).

<28> The composition according to clause <27> characterized in that one or more compound(s) according to group f) is/are one or more anionic surfactant(s), preferably selected from ethoxylated or non-ethoxylated alkyl ether sulfate surfactants, alkyl sulfates, ethoxylated and/or non-ethoxylated alkyl carboxylates, ethoxylated or non-ethoxylated amino acid surfactants, and/or their mixtures, and/or their salts, one or more alkyl sulfate(s) preferably ethoxylated alkyl ether sulfate surfactants or mixtures thereof, and/or salts thereof, having an alkyl chain length of C₁₀ to C₂₂ and an ethoxylation degree from 1 to 50, one or more non-ionic surfactant(s) selected from alkyl glycosides, alkyl polyglycosides, ethoxylated triglycerides, ethoxylated fatty alcohols, ethoxylated fatty acid esters, and/or their mixtures, one or more cationic surfactant(s) selected from quaternary ammonium surfactants having a carbon chain length in the range of C₁₂ to C₂₂ or surfactants having a tertiary amine group and at least one alkyl chain having a carbon chain length in the range of C₁₂ to C₂₂ such as alkylamidoalkylamine surfactants, and/or their salts, and/or their mixtures, and one or more amphoteric/zwitterionic surfactants of betaine type, and/or their salt(s), and/or their mixtures.

<29> The composition according to any of the clauses <27> to <28> characterized in that the total concentration of surfactants is in the range of 0.1% to 10% by weight, calculated to the total weight of composition A.

<30> The composition according to any of the clauses <1> to <29> characterized in that it comprises one or more thickening polymer(s), preferably one or more thickening polymer(s) selected from non-ionic thickening polymers and/or anionic thickening polymers, and/or their mixtures.

<31> The composition according to clause <30> characterized in that one or more thickening polymer(s) is/are a non-ionic thickening polymer, preferably a cellulose-based polymers, more preferably methylcellulose, hydroxymethylcellulose, hydroxyethylcellulose, hydroxypropylcellulose, hydroxyethyl-methylcellulose, and alkylated hydroxyl celluloses such as (C₂-C₈)-alkylcelluloses or cetyl hydroxyethylcellulose, an anionic thickening polymer, preferably a naturally-based anionic polymers and/or synthetic anionic polymers, more preferably xanthan gum, dehydroxanthan gum, hydroxypropylxanthan gum, carboxymethyl cellulose and starch based polymers such as vegetable starch and/or their synthetically modified derivatives such as hydroxypropyl starch phosphate, alginic acids, sodium alginates, ammonium alginates, calcium alginates, gum arabic, and guar gum, and/or an associative thickening polymer such as acrylates/steareth-30 methacrylate copolymer.

<32> The composition according to any of the clauses <30> to <31> characterized in that one or more thickening polymer is a natural anionic polymer, more preferably xanthan gum and/or dehydroxanthan gum.

<33> The composition according to any of the clauses <30> to <32> characterized in that the total concentration of thickening polymers is in the range of 0.1% to 15% by weight, preferably in the range of 0.25% to 12% by weight, more preferably in the range of 0.5% to 10% by weight, calculated to the total weight of composition A.

<34> The composition according to any of the clauses <1> to <33> characterized in that it comprises one or more oxidizing agent(s) being solid at 25°C and atmospheric pressure, more preferably one or more peroxide being solid at 25°C and atmospheric pressure, still more preferably it comprises a metal peroxide such as sodium peroxide, potassium peroxide, magnesium peroxide, melamine peroxide, urea peroxide, and/or their mixtures.

<35> The composition according to clause <34> characterized in that the total concentration of one or more oxidizing agent being solid at 25°C and atmospheric pressure is in the range of 0.1% to 50% by weight, more preferably in the range of 0.25% to 40% by weight, further more preferably in the range of 1% to 30% by weight, calculated to the total weight composition A.

The present disclosure is also directed to <36> a product for dyeing of keratin fibers, preferably of human keratin fibers, more preferably of human hair, comprising
- a composition A as defined in any of the clauses <1> to <35>,
- a composition B, preferably comprising one or more oxidizing agent(s).

<37> The product according to clause <36> characterized in that composition B is an aqueous composition having a pH in the range of 1 to 6, preferably in the range of 1.5 to 5, more preferably in the range of 2 to 4.5, and preferably comprises one or more oxidizing agent(s), more preferably hydrogen peroxide, still more preferably it comprises hydrogen peroxide at a concentration in the range of 10.25% to 30% by weight, more preferably in the range of 0.5% to 25% by weight, still more preferably in the range of 1% to 25% by weight, calculated to the total weight of composition B.

<38> The product according to any of the clauses <36> to <37> characterized in that composition B comprises one or more alkylamine salt(s) or alkanolamine salt(s), preferably according to the following general structure 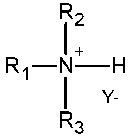 wherein R₁, R₂, and R₃ are independently selected from H, linear C₁-C₆ alkyl which may be substituted with one hydroxyl group, or branched C₃-C₁₂ alkyl or alkanol, wherein at least one of R₁, R₂, or R₃ is different from H, and Y- is an anion.

<39> The product according to clause <38> characterized in that the anion Y- is sulfate, hydrochloride, chloride, hydrobromide, bromide, nitrate, phosphate, hydrogen phosphate, dihydrogen phosphate, methosulfate, citrate, succinate, tartrate, lactate, tosylate, benzoate, benzenesulfonate, acetate, and/or their mixtures

<40> The product according to any of the clauses <38> to <39> characterized in that one or more alkylamine salt(s) or alkanolamine salt(s) is/are salts of mono- and/or diethanolamine, butyl ethanolamine, butyl diethanolamine, dibutyl ethanolamine, methylethanolamine, triethanolamine, N-lauryl diethanolamine, diisopropanolamine, dimethyl isopropanolamine, isopropanolamine, triisopropanolamine, isobutanolamine, tris-(hydroxymethyl)-aminomethane, and/or their mixtures, more preferably it/they is/are mono- and/or diethanolamine salt(s), and/or their mixtures.

<41> The product according to any of the clauses <38> to <40> characterized in that the total concentration of alkylamine salt(s) or alkanolamine salt(s) in composition B is in the range 0.1% to 15% by weight, preferably in the range of 0.2% to 12% by weight, more preferably in the range of 0.5% to 10% by weight, more preferably in the range of 0.5% to 8% by weight, calculated to the total weight of composition B.

<42> The product according to any of the clauses <38> to <41> characterized in that composition B is an emulsion, thickened gel, or a combination thereof, and preferably comprises one or more lipophilic compound(s) according to group d) and/or one or more surfactant(s) as compound(s) according to group f) and/or one or more thickening polymer(s).

<43> The product according to any of the clauses <38> to <42> characterized in that the total concentration of compounds according to group d) in composition B is in the range of 1% to 20% by weight, preferably in the range of 2% to 15% by weight, more preferably in the range of 3% to 12% by weight, calculated to the total weight of composition B.

<44> The product according to any of the clauses <38> to <43> characterized in that the total concentration of one or more compound(s) according to f) in composition B is in the range of 0.1% to 10% by weight, calculated to the total weight of composition B.

<45> The product according to any of the clauses <38> to <44> characterized in that the total concentration of thickening polymers in composition B is in the range of 0.1% to 15% by weight, preferably in the range of 0.25% to 12% by weight, more preferably in the range of 0.5% to 10% by weight, calculated to the total weight of composition B.

<46> The product according to any of the clauses <38> to <45> characterized in that composition B has a viscosity in the range of 1,000 Pas to 25,000 mPas, preferably 2,000 mPas to 20,000 mPas, more preferably in the range of 2,500 mPas to 17,500 mPas, determined by cone plate viscometry at 25°C under atmospheric conditions, suitably with Brookfield viscometer with spindle #4.

<47> The product according to any of the clauses <38> to <46> characterized in that the mixing ratio by weight of compositions A and B is in the range of 1:1 to 1:20, preferably in the range of 1:2 to 1:15, more preferably in the range of 1:4 to 1:12, still more preferably in the range of 1:5 to 1:10.

The present disclosure is also directed to <48> a method for dyeing keratin fibers, preferably human keratin fibers, more preferably human hair, comprising the steps of:
i) mixing composition A as defined in any of the clauses <1> to <35> with an aqueous composition, preferably with composition B as defined in any of the clauses <36> to <47> to yield a ready-to-use composition having a pH in the range of 7 to 12,
ii) applying the composition A or the ready-to-use composition onto keratin fibers and leaving it for a time period in the range of 1 min to 60 min,
iii) optionally rinsing-off the keratin fibers and optionally drying the keratin fibers.

<49> The method according to clause <48> characterized in that the ready-to-use composition is left for a time period of 1 min to 60 min as defined in step ii), preferably for a time period of 5 min to 45 min, more preferably for a time period of 10 min to 35 min.

<50> The method according to any of the clauses <48> to <49> characterized in that heat may be applied while leaving the composition A or the ready-to-use composition onto keratin fibers, preferably at a temperature in the range of 30°C to 50°C.

<51> The method according to any of the clauses <48> to <50> characterized in that compositions A and B of step i) are mixed, preferably at a mixing ratio in the range of 1:1 to 1:20, preferably in the range of 1:2 to 1:15, more preferably in the range of 1:4 to 1:12, still more preferably in the range of 1:5 to 1:10.

The following examples are to illustrate the invention, but not to limit it.

### EXAMPLES

**Table 1**

| **Ingredients composition A** | **Inv. Ex. 1** | **Inv. Ex. 2** | **Comp. Ex. 1** | **Comp. Ex. 2** |
|---|---|---|---|---|
| | **[% by weight]** | | | |
| 1,4-diamino-2-methoxymethyl-benzene | 8.66 | 8.66 | 8.66 | 8.66 |
| 4-Chlororesorcinol | 5.0 | 5.0 | 5.0 | 5.0 |
| Trisodium phosphate | 50.0 | 50.0 | - | - |
| Sodium disilicate | - | - | 50.0 | 50.0 |
| Corn starch | Ad 100.0 | - | Ad 100.0 | - |
| Kaolin | - | Ad 100.0 | - | Ad 100.0 |
| | | | | |
| Mixing ratio by weight comp. A:B | 1:10 | 1:10 | 1:10 | 1:10 |
| | | | | |

| **Results** | | | | |
|---|---|---|---|---|
| Color of stored powder after 7 days | Off white | Light brown | Grey | Black |
| Manageability of stored powder after 7 days | Free-flowing | Free-flowing | Large agglom erates | Large agglom erates |

**Composition B**

| | **% by weight** |
|---|---|
| Hydrogen peroxide | 4.5 |
| Monoethanolamine sulfate | 2.0 |
| Phosphoric acid | ad pH 2.0 |
| Water | ad 100.0 |

The inventive compositions kept their cosmetic appearance as well as their manageability over storage, whereas the comparative compositions appeared discolored and agglomerated.

Compositions A and B were then mixed in a weight ration of A:B of 1:10 to prepare the ready-to-use mixture directly prior to application onto bleached human keratin fibers. The mixture was then applied onto keratin fibers and left for 30 min at 40°C. Then the hair streaks were rinsed-off. As a result, the inventive compositions delivered an insensitive color result, while the comparative compositions showed less intensity, judged by the naked human eye.

### Methods

### Compositions and their storage

The compositions of table 1 were prepared by conventional mixing methods in an aluminum ball mill.

Compositions were then stored at 80% relative humidity and 24°C for 7 days. Powder color was judged by the naked human eye.

Manageability was investigated by pouring the composition into another vessel and observing it with the naked human eye.

The following examples are within the scope of the present invention.

### Inventive Example 3

**Composition A**

| | **% by weight** |
|---|---|
| 1,4-diamino-2-methoxymethyl-benzene sulfate | 0.097 |
| 5-Am ino-6-chloro-o-cresol | 0.1 |
| HC Blue 18 | 0.05 |
| HC Red 18 | 0.08 |
| HC Yellow 16 | 0.02 |
| Trisodium phosphate | 50.0 |
| Corn starch | ad 100.0 |

Composition B is as disclosed under table 1. Mixing weight ratio of compositions A:B is 1:10.

### Inventive Example 4

**Composition A**

| | **% by weight** |
|---|---|
| HC Blue 18 | 0.05 |
| HC Red 18 | 0.08 |
| HC Yellow 16 | 0.02 |
| Trisodium phosphate | 50.0 |
| Corn starch | ad 100.0 |

Composition B is as disclosed under table 1. Mixing weight ratio of compositions A:B is 1:10.

## Claims

1. A dyeing composition A for keratin fibers, preferably for human keratin fibers, more preferably for human hair, comprising
a) one or more hair dye(s), preferably one or more oxidative dye coupler(s) and/or oxidative dye precursor(s), and/or their salt(s), and/or their mixtures, and/or one or more direct dye(s), and/or their salt(s), and/or their mixtures,
b) one or more salt(s) according to the following structure
X₃PO₄
wherein X is a metal, and
wherein the total concentration of water is 10% by weight or less, calculated to the total weight of composition A.

2. The composition according to claim 1 **characterized in that** one or more compound(s) according to group a) is/are p-phenylenediamine, 1,4-diamino-2-methoxymethyl-benzene, p-aminophenol, 2,5-diamino-toluene, 2-N-propyl-p-phenylenediamine, 2-N-ethyl-p-phenylenediamine, 2,6-dimethyl-p-phenylene-diamine, 2-(2,5-diaminophenyl)-ethanol, 1-amino-4-bis-(2'-hydroxyethyl)-aminobenzene, 2-(2-hydroxyethylamino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene, 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 3,5-diamino-1,2,4-triazole, 4-aminophenol and the derivatives thereof such as 4-amino-3-methylphenol, 2-chloro-4-aminophenol, 2,6-dichloro-4-aminophenol, 2,4-diamino-phenol, 2,6-dibromo-4-aminophenol, tetraamino pyrimidines, triaminohydroxy pyrimidines, diaminomono- and -dihydroxy pyrimidines, aminotriazines, 5-amino salicylic acid and/or 1,2,4-triamino benzene, 2,5-diaminopyridine, 2,3-diaminopyridine, 2,6-diaminopyridine, 3-amino-2-methyl amino-6-methoxypyridine, 2-dimethyl-5-aminopyridine, 2-dimethyl aminoethyl-3-hydroxypyridine, 2-amino-4,6-dimethyl pyridine, 2-amino-3-hydroxypyridine, 3-amino-2-(β-hydroxyethyl amino)-6-methoxypyridine, 2,6-dimethyl amino-5-aminopyridine, 2-di(hydroxyethyl) amino-5-aminopyridine, 2-hydroxyethylamino-5-aminopyridine, 4-hydroxy-2,5,6-triaminopyrimidine, and/or their salt(s), and/or their mixtures, preferably one or more compound(s) according to group a) is/are 1,4-diamino-2-methoxymethyl-benzene, and/or its salt(s).

3. The composition according to any of the preceding claims **characterized in that** one or more compound(s) according to group a) is/are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9, Disperse Violet 1, Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic Red 51, Basic Yellow 87, HC Blue 17 and Basic Orange 31, Basic Yellow 87, Basic Orange 31, HC Blue 17, Basic Blue 124, HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Blue 18, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Red 18, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No. 11, HC Yellow No.12, HC Yellow No.13, HC Yellow No.14, HC Yellow No.15, HC Yellow 16, 2- Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol, 2-hydroxyethylpicramic acid, an/or their salt(s), and/or their mixtures, preferably one or more compound(s) according to group a) is/are HC Blue 18, HC Red 18, HC Yellow 16, Disperse Black 9, Acid Yellow 1, 2-amino-6-chloro-4-nitrophenol, and/or their salt(s), and/or their mixtures.

4. The composition according to any of the preceding claims **characterized in that** the total concentration of compound(s) according to group a), preferably the total concentration of 1,4-diamino-2-methoxymethyl-benzene, and/or its salt(s), is in the range of 0.01% to 65% by weight, preferably in the range of 0.02% to 50% by weight, more preferably in the range of 0.03% to 30% by weight, more preferably in the range of 0.03% to 20% by weight, calculated to the total weight of composition A.

5. The composition according to any of the preceding claims **characterized in that** X for compound(s) according to group b) is an alkaline or earth alkaline metal, preferably X is sodium or potassium, more preferably it is sodium.

6. The composition according to any of the preceding claims **characterized in that** compound(s) according to group b) is/are anhydrous compounds.

7. The composition according to any of the preceding claims **characterized in that** the total concentration of compound(s) according to group b), preferably the total concentration of trisodium phosphate and/or trispotassium phosphate, is in the range of 1% to 80% by weight, preferably in the range of 2% to 70% by weight, more preferably in the range of 5% to 65% by weight, calculated to the total weight of the composition A.

8. The composition according to any of the preceding claims **characterized in that** the total concentration of water in composition A is 5% by weight or less, preferably 1% by weight or less, more preferably 0.1% by weight or less, calculated to the total weight of composition A, still more preferably composition A is an anhydrous composition.

9. The composition according to any of the preceding claims **characterized in that** composition A comprises one or more pulverulent excipients, preferably one or more organic or inorganic pulverulent excipient, more preferably one or more pulverulent excipient selected from diatomaceous earth, kaolin, bentonite, silicates, starches such as corn starch, tapioca starch, rice starch, wheat starch and potato starch, nylon powder, montmorillonite, gypsum, sawdust and perlite, and/or their mixtures.

10. The composition according to any of the preceding claims **characterized in that** the composition is a solid composition at 25°C and atmospheric pressure, preferably a powder composition or a pellet composition or a tablet composition or a capsule composition, more preferably a hair dyeing powder composition or a hair dyeing pellet composition or a hair dyeing tablet composition, still more preferably it is a hair dyeing powder composition.

11. The composition according to any of the preceding claims **characterized in that** the composition is a paste-like composition, preferably having a viscosity in the range of 10,000 mPas to 500,000 mPas, more preferably in the range of 20,000 mPas to 400,000 mPas, still more preferably in the range of 30,000 mPas to 250,000 mPas, measured by cone-plate viscometry at 25°C and atmospheric pressure.

12. The composition according to any of the preceding claims **characterized in that** it comprises one or more oxidizing agent being solid at 25°C and atmospheric pressure, preferably percarbonates, borates, perborates, melamine peroxide, urea peroxide, and/or their salt(s), and/or their mixtures.

13. A product for dyeing of keratin fibers, preferably of human keratin fibers, more preferably of human hair, comprising
- a composition A as defined in any of claims 1 to 12,
- a composition B, preferably comprising one or more oxidizing agent(s).

14. The product according to claim 13 **characterized in that** composition B is an aqueous composition having a pH in the range of 1 to 6, preferably in the range of 1.5 to 5, more preferably in the range of 2 to 4.5, and preferably comprises one or more oxidizing agent(s), more preferably hydrogen peroxide, still more preferably it comprises hydrogen peroxide at a concentration in the range of 0.25% to 30% by weight, more preferably in the range of 0.5% to 25% by weight, still more preferably in the range of 1% to 25% by weight, calculated to the total weight of composition B.

15. The product according to any of the claims 13 to 14 **characterized in that** composition B comprises one or more alkylamine salt(s) or alkanolamine salt(s), preferably according to the following general structure 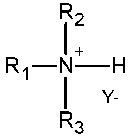 wherein R₁, R₂, and R₃ are independently selected from H, linear C₁-C₆ alkyl which may be substituted with one hydroxyl group, or branched C₃-C₁₂ alkyl or alkanol, wherein at least one of R₁, R₂, or R₃ is different from H, and Y- is an anion.

16. A method for dyeing keratin fibers, preferably human keratin fibers, more preferably human hair, comprising the steps of:
i) mixing composition A as defined in any of the claims 1 to 12 with an aqueous composition, preferably with composition B as defined in any of the claims 13 to 15 to yield a ready-to-use composition having a pH in the range of 7 to 12,
ii) applying the ready-to-use composition onto keratin fibers and leaving it for a time period in the range of 1 min to 60 min,
iii) optionally rinsing-off the keratin fibers and optionally drying the keratin fibers.
